# EUROPEAN PATENT APPLICATION

(11) **EP 4 803 087 A1**
(43) Date of publication of application: **09.09.2026**
(21) Application number: 24885730.2
(22) Date of filing: 30.10.2024
(51) Int. Cl.: A61K 31/7068, A61K 9/127, A61P 35/00, B01J 13/22

(54) **METHOD FOR PRODUCING LIPOSOME COMPOSITION, AND LIPOSOME COMPOSITION**

(30) Priority: 31.10.2023 JP 2023186240
(71) Applicant: FUJIFILM Corporation, Tokyo 106-8620 (JP)
(72) Inventor: MORI, Mikinaga, Ashigarakami-gun, Kanagawa 258-8577 (JP)
(74) Representative: Hoffmann Eitle
(86) International application number: PCT/JP2024/038601
(87) International publication number: WO 2025/094963

(57) **Abstract**

An object of the present invention is to provide a method for producing a liposome composition, which makes it possible to efficiently produce a liposome composition containing gemcitabine hydrochloride on a large scale, and a liposome composition. According to the present invention, there is provided a method for producing a liposome composition, the liposome composition containing a liposome containing an inner water phase and gemcitabine hydrochloride in a dissolved state, and an outer water phase which is an aqueous solution dispersing the liposome, the method: including steps (a) to (e) below. (a) a step of adding gemcitabine hydrochloride to water, raising a temperature to a range of 50°C or higher and 60°C or lower, stirring, and adjusting a pH to a range of 3.2 to 4.6 to prepare a dissolution solution of the gemcitabine hydrochloride; (b) a step of adding sodium chloride and lowering the temperature to lower than 50°C; (c) a step of mixing the dissolution solution of the gemcitabine hydrochloride with a dispersion liquid of an empty liposome; (d) a step of adjusting the pH to a range of 6.8 to 7.8; and (e) a step of heating to 55°C or higher.

## Description

### Technical Field

The present invention relates to a method for producing a liposome composition encompassing gemcitabine, and a liposome composition produced by the method for producing a liposome composition.

### Background Art

A liposome pharmaceutical product is a liposome encompassing a certain amount of an active pharmaceutical ingredient, the liposome consisting of a molecular double membrane of a lipid. The liposome pharmaceutical product can be suitably used, for example, as an anticancer agent. With the liposome pharmaceutical product, it is possible to expect reduction of toxicity specific to an anticancer agent, improvement of accumulation in a tumor, and improvement of drug efficacy by release control.

In the liposome, an amount ratio between the lipid constituting the shell of the liposome and the active pharmaceutical ingredient is an important element. For example, in a case where an excessive amount of the active pharmaceutical ingredient is packed into the shell of the liposome, the shell is deformed or destroyed. In addition, in a case where the amount of the active pharmaceutical ingredient is too small, a remarkable problem such as a large part of the active pharmaceutical ingredient encompassed in the liposome is rapidly released to the outside occurs. Therefore, it is important to manage the constituent concentrations of the lipid and the active pharmaceutical ingredient from the production to the completion. The production process of the liposome pharmaceutical product includes a large number of steps such as preparation of an empty liposome, preparation of a raw drug solution, mixing of the liposome and the raw drug solution, removal of the active pharmaceutical ingredient that cannot be encompassed in the liposome, dilution and concentration of the entire solution, and sterile filtration. During this period, it is important to manage the concentrations of the lipid and the active pharmaceutical ingredient, and particularly, it is important to completely dissolve the added active pharmaceutical ingredient.

As a method of loading the active pharmaceutical ingredient into the empty liposome in the production of the liposome, there is remote loading in which the added active pharmaceutical ingredient is actively diffused and encompassed inside the liposome, and passive loading in which the concentration gradient of the active pharmaceutical ingredient inside and outside the liposome is substantially the same.

Patent Document 1 discloses a method for producing a liposome composition encompassing a nucleic acid analog anticancer agent, the method including (a) a step of mixing a solution in which empty liposomes are dispersed with a nucleic acid analog anticancer agent solution, and (b) a step of heating the mixture to 55°C or higher.

Specifically, Patent Document 1 describes that 3.26 g of gemcitabine hydrochloride, 13.59 g of PBS, 19.05 g of water for injection, and 0.68 mL of 8 M sodium hydroxide are mixed and dissolved at 70°C to prepare a drug solution, and loading is performed using 15.7 mL of this drug solution.

### Prior Art Documents

### Patent Documents

Patent Document 1: WO2017/078009A

### Summary of Invention

### Object to be solved by the invention

In the passive loading, in order to increase the active pharmaceutical ingredient ratio in the final preparation, the concentration of the active pharmaceutical ingredient solution to be adjusted for the loading process is often increased to the limit of solubility, and the degree of difficulty in setting the production conditions is high. Gemcitabine cannot be subjected to remote loading due to the characteristics of the compound, and is produced by passive loading to obtain a satisfactory liposome pharmaceutical product. Since a compound such as gemcitabine has high pharmacological activity (toxicity), and the risk of exposure increases as the production scale increases, more stringent handling in a sealed environment is required. Therefore, as the production scale increases, it becomes difficult to confirm the progress of dissolution of the active pharmaceutical ingredient, and the degree of difficulty in setting the production conditions is increased.

In the method described in Patent Document 1, it takes time to completely dissolve the gemcitabine hydrochloride during production at a large scale intended for commercial production, and thus there has been a demand for a new production method for efficiently performing large-scale production. An object of the present invention is to provide a method for producing a liposome composition, which makes it possible to efficiently produce a liposome composition containing gemcitabine hydrochloride at a large scale. Another object of the present invention is to provide a liposome composition produced by the above-described method for producing a liposome composition.

### Means for solving the object

As a result of intensive studies to achieve the above-described object, the present inventors have found a method in which gemcitabine hydrochloride can be completely dissolved in a short time even at a large scale, and a liposome composition can be efficiently produced, thereby completing the present invention.

That is, according to the present invention, the following inventions are provided.
<1> A method for producing a liposome composition, the liposome composition containing a liposome containing an inner water phase and gemcitabine hydrochloride in a dissolved state, and an outer water phase which is an aqueous solution dispersing the liposome, the method comprising: steps (a) to (e) below:
   step (a) of preparing a dissolution solution of gemcitabine hydrochloride, the step including steps (1) to (3) below;
   step (1) of adding gemcitabine hydrochloride to water,
   step (2) of raising a temperature of the mixture obtained in the step (1) within a range of 50°C or higher and 60°C or lower and stirring the mixture, and
   step (3) of adding an alkali to the mixture obtained in the step (2) to adjust a pH to within a range of 3.2 to 4.6,
   step (b) of adding sodium chloride to the dissolution solution of gemcitabine hydrochloride obtained in the step (a), and a step of lowering the temperature of the obtained mixture to less than 50°C;
   step (c) of mixing the dissolution solution of gemcitabine hydrochloride obtained in the step (b) with a dispersion liquid of empty liposomes;
   step (d) of adding an alkali to the dissolution solution of the gemcitabine hydrochloride obtained in the step (c) and adjusting a pH to be in a range of 6.8 to 7.8; and
   step (e) of heating the dissolution solution obtained in the step (d) to 55°C or higher.
<2> The method for producing a liposome composition according to <1>, in which the step (a) is a step of preparing a gemcitabine hydrochloride solution in which gemcitabine hydrochloride is completely dissolved.
<3> The method for producing a liposome composition according to <1>, in which the step (a) is a step of preparing a gemcitabine hydrochloride solution in which the gemcitabine hydrochloride is completely dissolved by completion of step (2).
<4> The method for producing a liposome composition according to <1>, in which a stirring condition in the step (2) of the step (a) is such that a direction of a flow of a liquid generated by a stirring blade is a direction from a stirring blade portion toward an upper portion of a stirring blade shaft, and a circumferential speed of stirring is 10 to 300 m/min.
<5> The method for producing a liposome composition according to any one of <1> to <4>, further including, step (f) of removing gemcitabine hydrochloride present in the outer water phase after the step (e).
<6> The method for producing a liposome composition according to any one of <1> to <4>, in which in the step of adding gemcitabine hydrochloride to water in the step (1) of the step (a), an addition amount of water is 5 times or more and 15 times or less the gemcitabine hydrochloride.
<7> The method for producing a liposome composition according to any one of <1> to <4>, in which in the step of mixing the dissolution solution of the gemcitabine hydrochloride obtained in the step (b) with the dispersion liquid of the empty liposome in the step (c), an amount of water in the dispersion liquid is 5 times or more and 15 times or less an amount of the gemcitabine hydrochloride.
<8> A liposome composition obtained by a production method comprising: the steps (a) to (e) below, in which the liposome composition includes liposome having an inner water phase and an aqueous solution constituting an outer water phase in which the liposomes are dispersed, and the liposomes encapsulate gemcitabine hydrochloride in a dissolved state.
   step (a) of preparing a dissolution solution of gemcitabine hydrochloride, the step including steps (1) to (3) below;
   step (1) of adding gemcitabine hydrochloride to water,
   step (2) of raising a temperature of the mixture obtained in the step (1) within a range of 50°C or higher and 60°C or lower and stirring the mixture, and
   step (3) of adding an alkali to the mixture obtained in the step (2) to adjust a pH to within a range of 3.2 to 4.6,
   step (b) of adding sodium chloride to the dissolution solution of gemcitabine hydrochloride obtained in the step (a), and a step of lowering the temperature of the obtained mixture to less than 50°C;
   step (c) of mixing the dissolution solution of gemcitabine hydrochloride obtained in the step (b) with a dispersion liquid of empty liposomes;
   step (d) of adding an alkali to the dissolution solution of the gemcitabine hydrochloride obtained in the step (c) and adjusting a pH to be in a range of 6.8 to 7.8; and
   step (e) of heating the dissolution solution obtained in the step (d) to 55°C or higher.
<9> The liposome composition according to <8>, in which the step (a) is a step of preparing a gemcitabine hydrochloride solution in which the gemcitabine hydrochloride is completely dissolved.
<10> The liposome composition according to <8>, in which the step (a) is a step of preparing a gemcitabine hydrochloride solution in which the gemcitabine hydrochloride is completely dissolved by completion of step (2).
<11> The liposome composition according to <8>, in which a stirring condition in the step (2) of the step (a) is such that a direction of a flow of a liquid generated by a stirring blade is a direction from a stirring blade portion toward an upper portion of a stirring blade shaft, and a circumferential speed of stirring is 10 to 300 m/min.
<12> The liposome composition according to any one of <8> to <11>, further including, step (f) of removing gemcitabine hydrochloride present in the outer water phase after the step (e).
<13> The liposome composition according to any one of <8> to <11>, in which in the step (1) of the step (a) of adding gemcitabine hydrochloride to water, an amount of water added is 5 times or more and 15 times or less an amount of the gemcitabine hydrochloride.
<14> The liposome composition according to any one of <8> to <11>, in which in the step of mixing the dissolution solution of the gemcitabine hydrochloride obtained in the step (b) with the dispersion liquid of the empty liposome in the step (c), an amount of water in the dispersion liquid is 5 times or more and 15 times or less an amount of the gemcitabine hydrochloride.

### Effect of the invention

According to the present invention, it is possible to produce liposomes having a desired gemcitabine concentration and a desired lipid concentration without leaving undissolved gemcitabine in the middle of the production. According to the present invention, it is possible to efficiently produce a liposome composition on a large scale.

### Embodiments for carrying out the invention

In the present specification, the numerical ranges shown using "to" indicate ranges including the numerical values described before and after "to" as the minimum value and the maximum value.

In the present invention, unless otherwise specified, "%" means a mass percentage.

In the present specification, in a case where a plurality of substances corresponding to each component in a composition, unless otherwise specified, the amount of each component in the composition means the total amount of the plurality of substances present in the composition.

The "empty liposome" means a liposome that does not encompass a drug.

The "leakage" means that a drug encompassed in a liposome passes through a lipid membrane constituting the liposome and comes out (is released) to the outside of the liposome.

The "leakage rate" means an amount of a drug encompassed in a liposome that passes through a lipid membrane constituting the liposome and comes out to the outside of the liposome per unit time. It is important to maintain a state in which a drug is encompassed in a liposome during the production and storage of the liposome before administration, and it is preferable to suppress the leakage rate to be low. On the other hand, in the blood after administration, it is preferable to leak a drug at a certain rate in order to expose a cancer tumor to the drug.

The term "retention in the blood" means a property in which a drug in a state of being encapsulated in a liposome is present in the blood in a subject to which a liposome composition has been administered.

The "water phase" means an outer water phase and an inner water phase. The "outer water phase" means an aqueous solution in which a liposome is dispersed. For example, in a case of an injection, a solution occupying the outside of the liposome of a dispersion liquid of liposomes packaged and stored in a vial or prefilled syringe becomes an outer water phase. In addition, similarly for a liquid to be dispersed at the time of use in a case of being administered by means of an attached liquid for dispersion or other dissolution liquid, a solution occupying the outside of the liposome of a dispersion liquid of liposomes becomes an outer water phase. The "inner water phase" means a water phase in a closed vesicle separated by a lipid bilayer of a liposome.

The "average particle diameter of a liposome" means a volume average particle diameter of a liposome present in a liposome composition. The average particle diameter of a liposome contained in the liposome composition according to the embodiment of the present invention is measured using a dynamic light scattering method. Examples of commercially available determination devices using dynamic light scattering include a concentrated system particle size analyzer FPAR-1000 (manufactured by Otsuka Electronics Co., Ltd.), a Nanotrac UPA (manufactured by Nikkiso Co., Ltd.), and a Nanosizer (manufactured by Malvern Panalytical Ltd.).

The "subject" is a mammal such as a human, a mouse, a monkey, or a domestic animal, which requires prevention or treatment thereof, and is preferably a human who requires prevention or treatment thereof.

Examples of the "tumor" include breast cancer, uterine corpus cancer, ovarian cancer, prostate cancer, lung cancer, gastric (gastric gland) cancer, non-small cell lung cancer, pancreatic cancer, head and neck squamous cell carcinoma, esophageal cancer, bladder cancer, melanoma, colorectal cancer, renal cell carcinoma, non-Hodgkin's lymphoma, and urinary tract urothelial carcinoma.

Hereinafter, the present invention will be specifically described.

A first aspect of the present invention is a method for producing a liposome composition, which is a method for producing a liposome composition containing a liposome containing an inner water phase and gemcitabine hydrochloride in a dissolved state, and an outer water phase which is an aqueous solution dispersing the liposome, the method including the following steps (a) to (e).

A second aspect of the present invention is a liposome composition obtained by a production method including the following steps (a) to (e), in which the liposome composition contains a liposome having an inner water phase, and an aqueous solution dispersing the liposome, which constitutes an outer water phase, and the liposome encompasses gemcitabine hydrochloride in a dissolved state.
step (a) of preparing a dissolution solution of gemcitabine hydrochloride, the step including steps (1) to (3) below;
step (1) of adding gemcitabine hydrochloride to water,
step (2) of raising a temperature of the mixture obtained in the step (1) within a range of 50°C or higher and 60°C or lower and stirring the mixture, and
step (3) of adding an alkali to the mixture obtained in the step (2) to adjust a pH to within a range of 3.2 to 4.6,
step (b) of adding sodium chloride to the dissolution solution of gemcitabine hydrochloride obtained in the step (a), and a step of lowering the temperature of the obtained mixture to less than 50°C;
step (c) of mixing the dissolution solution of gemcitabine hydrochloride obtained in the step (b) with a dispersion liquid of empty liposomes;
step (d) of adding an alkali to the dissolution solution of the gemcitabine hydrochloride obtained in the step (c) and adjusting a pH to be in a range of 6.8 to 7.8; and
step (e) of heating the dissolution solution obtained in the step (d) to 55°C or higher.

### (Liposome)

A liposome is a closed small vesicle formed of a lipid bilayer membrane that is formed from a lipid, and an aqueous phase (an inner water phase) is included in the space of the closed small vesicle. The inner water phase contains water. The liposome is generally present in a state of being dispersed in an aqueous solution (an outer water phase) outside the closed vesicle. The liposome may be a single lamella (also referred to as a single-layer lamella or a unilamella, in which a bilayer membrane has a single structure) or a multilamella (also referred to as a multilamella, in which a structure of a large number of bilayer membranes having an onion-like shape). Each layer is partitioned by an aqueous layer), but in the present invention, from the viewpoint of safety and stability in medical applications, a liposome of a single lamella is preferable.

The form of the liposome is not particularly limited as long as the liposome can encompass a drug (in the present invention, gemcitabine hydrochloride in a dissolved state). The term "encompass" means that the drug is contained in the inner water phase and the membrane itself of the liposome. Examples thereof include a form in which a drug is encapsulated in a closed space formed of a membrane, a form in which a drug is encompassed in the membrane itself, and the like, and a combination thereof may be used.

The average particle diameter of the liposome is preferably 10 nm to 150 nm, more preferably 20 nm to 110 nm, and still more preferably 30 nm to 90 nm.

The liposome preferably has a spherical shape or a shape close thereto.

The component constituting the lipid bilayer of the liposome is selected from lipids. Any lipid soluble in a mixed solvent of a water-soluble organic solvent and an ester-based organic solvent can be used as the lipid. Examples of the lipids include phospholipids, lipids other than phospholipids, cholesterols, lysophospholipids, and derivatives thereof. These components may be composed of a single type or a plurality of types of components.

Examples of the phospholipids include natural or synthetic phospholipids such as phosphatidylcholine (lecithin), phosphatidylglycerol, phosphatidic acid, phosphatidylethanolamine, phosphatidylserine, phosphatidylinositol, sphingomyelin, and cardiolipin, and hydrogenated products thereof (for example, hydrogenated soybean phosphatidylcholine (HSPC)). Among these, a hydrogenated phospholipid such as hydrogenated soybean phosphatidylcholine or sphingomyelin is preferable, and hydrogenated soybean phosphatidylcholine is more preferable. In the present invention, the term "phospholipid" also includes phospholipid derivatives obtained by modifying a phospholipid.

Examples of the lipids other than phospholipids include lipids containing no phosphoric acid, and examples thereof include glycerol lipids having no phosphoric acid moiety in the molecule and sphingolipids having no phosphoric acid moiety in the molecule. In the present invention, the term "lipid other than phospholipid" also includes derivatives of a lipid other than the phospholipid obtained by modifying a lipid other than phospholipid.

In a case where a compound having a basic functional group is bonded to a lipid, the lipid is referred to as a cationic lipid. The cationized lipid can modify, for example, the membrane of the liposome, and can enhance the adhesiveness to the cells which are target sites.

Examples of cholesterols include cholesterols which contain cyclopentahydrophenanthrene as a basic skeleton and in which carbon atoms are partially or completely hydrogenated and derivatives thereof. Examples thereof include cholesterol. In a case where the average particle diameter is reduced to 100 nm or less, the curvature of the lipid membrane increases. Since the strain of the membrane arranged in the liposome also increases, the water-soluble drug is more likely to leak out. As a means for suppressing the leakiness, it is effective to add cholesterol or the like in order to fill the strain of the membrane due to the lipid (membrane stabilization effect).

The addition of cholesterol to the liposome is expected to lower the fluidity of the membrane of the liposome, for example, by filling the gaps in the membrane of the liposome. In general, in the liposome, the amount of the cholesterols is usually desired to be included in an amount of about 50 mol% or less in the total (total lipid) mol of the lipid component.

The content of the cholesterols with respect to the total amount of the lipids constituting the liposome according to the embodiment of the present invention is preferably 10 mol% to 35 mol%, more preferably 15 mol% to 25 mol%, and still more preferably 17 mol% to 21 mol%. By setting the content of the cholesterols with respect to the total amount of the lipid component of the liposome to 10 mol% to 35 mol%, it is possible to obtain a liposome composition capable of achieving both an excellent leakage rate and storage stability.

In addition to the foregoing components, a hydrophilic polymer or the like for improving retention in blood, fatty acid, diacetyl phosphate, or the like as a membrane structure stabilizer, or α-tocopherol or the like as an antioxidant may be added to the liposome. In the present invention, it is preferable not to include an additive such as a dispersion aid which is not recognized for use in intravenous injection in pharmaceutical applications, for example, a surfactant.

The liposome according to the embodiment of the present invention preferably contains a phospholipid, a lipid other than the phospholipid, and cholesterols. The lipid other than the phospholipid is preferably modified with a hydrophilic polymer.

Examples of the hydrophilic polymer include polyethylene glycols, polyglycerins, polypropylene glycols, polyvinyl alcohol, a styrene-maleic anhydride alternating copolymer, polyvinylpyrrolidone, and a synthetic polyamino acid. The hydrophilic polymers may be each used alone or in a combination of two or more kinds thereof.

Among these, from the viewpoint of retention in blood of the preparation, polyethylene glycols, polyglycerins, and polypropylene glycols are preferable, and polyethylene glycol (PEG), polyglycerin (PG), and polypropylene glycol (PPG) are more preferable. From the viewpoint of general-purpose properties and retention in blood, polyethylene glycol (PEG) is still more preferable.

The molecular weight of PEG is not particularly limited, but is 500 to 10,000 daltons, preferably 1,000 to 7,000 daltons, and more preferably 2,000 to 5,000 daltons.

In the liposome according to the embodiment of the present invention, it is preferable to use a lipid modified with PEG (PEG-modified lipid) together with the main lipid contained in the liposome. Examples of the PEG-modified lipid include 1,2-distearoyl-3-phosphatidylethanolamine-polyethylene glycol such as 1,2-distearoyl-3-phosphatidylethanolamine-PEG2000 (manufactured by NOF Corporation), 1,2-distearoyl-3-phosphatidylethanolamine-PEG5000 (manufactured by NOF Corporation), and distearoyl glycerol-PEG2000 (manufactured by NOF Corporation). These PEG-modified lipids may be added so as to be contained in an amount of 0.3% to 50% by mass, preferably 0.5% to 30% by mass, and more preferably 1% to 20% by mass with respect to the total amount of lipids.

In the liposome according to the embodiment of the present invention, a combination of hydrogenated soybean phosphatidylcholine (the main lipid contained in the liposome), 1,2-distearoyl-3-phosphatidylethanolamine-polyethylene glycol (a lipid used in combination with the main lipid), and cholesterol is preferable.

In the liposome according to the embodiment of the present invention, it is preferable that the liposome does not contain an anionic polymer (polyanion).

### (Gemcitabine hydrochloride)

The liposome in the present invention encompasses gemcitabine hydrochloride as a drug.

Gemcitabine hydrochloride is an anticancer agent that has a structure similar to a substance that is a material of a nucleic acid such as a pyrimidine base and exhibits drug efficacy by being incorporated into a DNA chain.

### (Gemcitabine hydrochloride encompassed in liposome)

The gemcitabine hydrochloride encompassed in the liposome in the present invention is present in a dissolved state in the inner water phase of the liposome. Here, the gemcitabine hydrochloride is regarded as being encompassed in a dissolved state in a case where the amount of the gemcitabine hydrochloride filled in the liposome is equal to or less than the saturated solubility of the gemcitabine hydrochloride in the composition liquid of the inner water phase with respect to the volume of the liposome. In addition, even in a case where the saturated solubility or more, the drug crystal is not observed by Cryo-TEM or the diffraction pattern due to the crystal lattice is not observed by the XRD measurement, most of the gemcitabine hydrochloride encompassed in the liposome is dissolved, and the gemcitabine hydrochloride is regarded as being encompassed in a dissolved state. In this case, it is presumed that the dissolution is promoted by the physicochemical environment created by the lipid membrane, or a part of the drug is incorporated into the lipid membrane. In addition, the gemcitabine hydrochloride encompassed by a method of forming a solid substance inside the liposome and encapsulating the gemcitabine hydrochloride is not in a dissolved state as referred to in the present invention.

The content of the gemcitabine hydrochloride encompassed in the liposome is preferably 0.1 to 2.0 mg/mL and more preferably 0.2 to 1.0 mg/mL with respect to the liposome composition.

### (Gemcitabine hydrochloride/lipid ratio)

The "gemcitabine hydrochloride/lipid ratio" means a mass ratio of the gemcitabine hydrochloride encompassed in the liposome to the lipid constituting the liposome.

The gemcitabine hydrochloride/lipid ratio is preferably 2% by mass to 10% by mass, more preferably 2.5% by mass to 10% by mass, and still more preferably 3% by mass to 10% by mass. By setting the gemcitabine hydrochloride/lipid ratio to 2% by mass to 10% by mass, it is possible to suppress hyperlipidemia and fatty liver due to the lipid while controlling the leakage rate within a preferred range. The lipid in the gemcitabine hydrochloride/lipid ratio means all the lipids constituting the liposome, and the lipid also includes lysophospholipid.

### (Liposome composition)

The liposome composition according to the embodiment of the present invention contains a liposome containing an inner water phase and gemcitabine hydrochloride in a dissolved state, and an outer water phase which is an aqueous solution dispersing the liposome.

From the viewpoint of filter filtration suitability suitable for commercial production and tumor accumulation due to the EPR effect, the liposome composition preferably contains 1% by mass or less of liposomes having a particle diameter of 200 nm or more, more preferably 0.5% by mass or less, and still more preferably 0.1% by mass or less.

In the liposome composition, the osmotic pressure of the inner water phase of the liposome is preferably 2 times to 8 times, more preferably 2.5 times to 6 times, and still more preferably 2.5 times to 5 times the osmotic pressure of the outer water phase of the liposome. By setting the osmotic pressure of the inner water phase of the liposome to be 2 times to 8 times the osmotic pressure of the outer water phase of the liposome, it is possible to achieve both an excellent drug leakage rate and storage stability.

An aqueous solvent, an additive, and the like can be appropriately added to the liposome composition. In connection with the route of administration, the liposome composition according to the embodiment of the present invention may also contain at least one of an isotonizing agent, a stabilizer, an antioxidant, or a pH adjusting agent which is pharmaceutically acceptable.

The isotonizing agent is not particularly limited and examples thereof include inorganic salts such as sodium chloride, potassium chloride, sodium hydrogen phosphate, sodium dihydrogen phosphate, and potassium dihydrogen phosphate; polyols such as glycerol, mannitol, and sorbitol; and sugars such as glucose, fructose, lactose, and sucrose.

The stabilizer is not particularly limited and examples thereof include sugars such as glycerol, mannitol, sorbitol, lactose, and sucrose.

The antioxidant is not particularly limited and examples thereof include ascorbic acid, uric acid, tocopherol homologues (for example, vitamin E, four tocopherol isomers α, β, γ, and δ), cysteine, and ethylenediaminetetraacetic acid (EDTA). Stabilizers and antioxidants may be respectively used alone or in combination of two or more thereof.

Examples of the pH adjusting agent include sodium hydroxide, citric acid, acetic acid, triethanolamine, sodium hydrogen phosphate, sodium dihydrogen phosphate, and potassium dihydrogen phosphate.

The liposome composition according to the embodiment of the present invention may contain an organic solvent, collagen, polyvinyl alcohol, polyvinyl pyrrolidone, a carboxyvinyl polymer, sodium carboxymethyl cellulose, sodium polyacrylate, sodium alginate, water-soluble dextran, sodium carboxymethyl starch, pectin, methyl cellulose, ethyl cellulose, xanthan gum, gum arabic, casein, gelatin, agar, diglycerin, propylene glycol, polyethylene glycol, vaseline, paraffin, stearyl alcohol, stearic acid, human serum albumin (HSA), mannitol, sorbitol, lactose, phosphate buffered saline (PBS), sodium chloride, sugars, a biodegradable polymer, a serum-free medium, each of which is pharmaceutically acceptable, or an additive which is acceptable as a pharmaceutical additive.

### (Leakage rate in blood)

In the liposome composition according to the embodiment of the present invention, the leakage rate of the drug in blood is preferably 10% by mass/24 hr to 70% by mass/24 hr, more preferably 20% by mass/24 hr to 60% by mass/24 hr, and still more preferably 20% by mass/24 hr to 50% by mass/24 hr.

Since the leakage rate in blood depends on the temperature, it is preferable to measure the leakage rate in blood under a constant temperature condition. For example, in the case of a human, the temperature is not particularly limited, but it is preferable to measure the leakage rate in blood within a range of body temperature (35°C to 38°C).

In a case where the leakage rate in blood is less than 10% by mass/24 hr, it is not possible to obtain a sufficient exposure time in the body as an anticancer agent, and the expected drug efficacy is not obtained in many cases. In addition, in some cases, the liposome containing gemcitabine hydrochloride remains in the body for an unnecessarily long time, and thus the liposome may accumulate in a tissue that is originally difficult to distribute, such as the skin, and unexpected toxicity may be exhibited. In addition, in a case where the leakage rate in blood is greater than 70% by mass/24 hr, the amount of the drug exposed per unit time increases, and thus the maximum blood concentration increases, which increases the toxicity. In addition, since the leaked drug is distributed to tissues other than the tumor portion and undergoes rapid metabolism, the retention in blood is decreased, which is not preferable.

A method of measuring the leakage rate in blood is not particularly limited, but the blood or blood plasma is collected from a mammalian animal or a model system every unit time after the administration to a target mammalian animal, a model system, or the like, and the pretreatment or the like is performed as necessary. Then, the target drug can be measured by a method such as a liquid chromatograph or a mass spectrum.

### (Method for producing liposome composition)

The method of producing a liposome composition according to the embodiment of the present invention includes the following steps (a) to (e).
step (a) of preparing a dissolution solution of gemcitabine hydrochloride, the step including steps (1) to (3) below;
step (1) of adding gemcitabine hydrochloride to water,
step (2) of raising a temperature of the mixture obtained in the step (1) within a range of 50°C or higher and 60°C or lower and stirring the mixture, and
step (3) of adding an alkali to the mixture obtained in the step (2) to adjust a pH to within a range of 3.2 to 4.6,
step (b) of adding sodium chloride to the dissolution solution of gemcitabine hydrochloride obtained in the step (a), and a step of lowering the temperature of the obtained mixture to less than 50°C;
step (c) of mixing the dissolution solution of gemcitabine hydrochloride obtained in the step (b) with a dispersion liquid of empty liposomes;
step (d) of adding an alkali to the dissolution solution of the gemcitabine hydrochloride obtained in the step (c) and adjusting a pH to be in a range of 6.8 to 7.8; and
step (e) of heating the dissolution solution obtained in the step (d) to 55°C or higher.

### <Step (a)>

The step (a) is a step of preparing a dissolution solution of gemcitabine hydrochloride, the step including the following steps (1) to (3).
step (1) of adding gemcitabine hydrochloride to water,
step (2) of raising a temperature of the mixture obtained in the step (1) within a range of 50°C or higher and 60°C or lower and stirring the mixture, and
step (3) of adding an alkali the mixture obtained in the step (2) and adjust the pH to a range of 3.2 to 4.6

In the step of adding gemcitabine hydrochloride to water in the step (1), an addition amount of water is preferably 5 times or more and 15 times or less the amount of gemcitabine hydrochloride, and more preferably 7 times or more and 13 times or less the amount of gemcitabine hydrochloride.

In the step (2), the mixture obtained in the step (1) is raised to a temperature in a range of 50°C or higher and 60°C or lower and agitated. In the agitation conditions in the step (2), it is preferable that a direction of a flow of a liquid generated by the stirring blade is a direction from the stirring blade portion toward an upper portion of the stirring blade shaft, and a circumferential speed of agitation is 10 to 300 m/min. The circumferential speed of agitation is more preferably 50 to 150 m/min.

Gemcitabine hydrochloride can be dissolved by raising the temperature and agitating in the step (2). The step (a) is preferably a step of preparing a gemcitabine hydrochloride solution in which gemcitabine hydrochloride is completely dissolved. The step (a) is more preferably a step of preparing a gemcitabine hydrochloride solution in which gemcitabine hydrochloride is completely dissolved by the step (2). The step (a) is a step of preparing a gemcitabine hydrochloride solution in which gemcitabine hydrochloride is completely dissolved by the end of the step (2).

A concentration of gemcitabine hydrochloride in the dissolution solution of gemcitabine hydrochloride is preferably 60 to 200 mg/mL, and more preferably 100 to 140 mg/mL.

As the alkali in the step (3), a general water-soluble base such as sodium hydroxide, potassium hydroxide, and calcium hydroxide can be used, but the alkali is not particularly limited. In a case where the pH in the step (3) is less than 3.2, there is a concern that the lipid constituting the liposome may be decomposed, and in a case where the pH is higher than 4.6, gemcitabine hydrochloride is less likely to be dissolved.

### <Step (b)>

The step (b) is a step of adding sodium chloride to the dissolution solution of gemcitabine hydrochloride obtained in the step (a), and a step of lowering the temperature of the obtained mixture to less than 50°C.

In the present invention, the step (a) is performed in a state in which sodium chloride is not present, and the gemcitabine hydrochloride solution in which gemcitabine hydrochloride is dissolved is prepared, and then sodium chloride is added in the step (b). By performing the step (a) in a state in which sodium chloride is not present, gemcitabine hydrochloride can be dissolved even on a large scale.

### <Step (c)>

The step (c) is a step of mixing the dissolution solution of gemcitabine hydrochloride obtained in the step (b) with a dispersion liquid of empty liposomes.

The dispersion liquid of empty liposomes can be produced by a method including emulsifying a lipid dissolved in an organic solvent.

In the emulsification step, an oil phase in which at least one kind of lipid is dissolved in an organic solvent and an aqueous phase are mixed, and the aqueous solution containing the lipid is stirred and emulsified. An oil phase where lipid has been dissolved in an organic solvent and a water phase are mixed, stirred, and emulsified to thereby prepare an emulsion where the oil phase and the water phase are emulsified in an O/W type (oil-in-water type). After mixing, liposomes are formed by removing a portion or all of the organic solvent derived from the oil phase by an evaporation step which will be described below. Alternatively, a portion or all of the organic solvent in the oil phase is evaporated in the course of the stirring-emulsification to form liposomes.

As a method of stirring, ultrasonic waves or mechanical shearing force is used for particle miniaturization. In addition, extruder processing or microfluidizer processing of allowing to pass through a filter having a certain pore size can be carried out for uniformity of particle sizes. Use of an extruder or the like can result in decomposition of secondarily formed multivesicular liposomes into univesicular liposomes. In the present invention, from the viewpoint of simplifying the production step, it is preferable that the empty liposomes are used in the next step without being subjected to the extrusion treatment.

By optionally selecting the stirring speed and time, the average particle diameter of the liposomes to be prepared can be controlled. From the viewpoint of obtaining liposomes having safety and stability, it is preferable to apply a shear of 20 m/sec or more in terms of circumferential speed to the aqueous solution containing the lipid. The shear is not limited, but specifically, it is preferable to apply a shear of 20 m/sec to 35 m/sec in terms of circumferential speed, and it is more preferable to apply a shear of 23 m/sec to 30 m/sec in terms of circumferential speed.

The emulsifying step is not limited as long as it is a step of emulsification, but it is preferably a step of applying a high shearing force and performing microparticulation with an emulsifying step including an organic solvent. If necessary, evaporation (desolvation) of the organic solvent used in the emulsifying step may be carried out to form liposomes.

The liquid temperature in the emulsifying step in a case of producing liposomes can be appropriately adjusted, but the liquid temperature at the time of mixing an oil phase and a water phase is preferably equal to or higher than a phase transition temperature of the lipid to be used. For example, in a case where a lipid having a phase transition temperature of 35°C to 40°C is used, the liquid temperature at the time of mixing an oil phase and a water phase is preferably 35°C to 70°C.

### (Oil phase)

As the organic solvent used as the oil phase, a mixed solvent of a water-soluble organic solvent and an ester-based organic solvent is used. In the present invention, it is preferable that the organic solvent is substantially not used, and it is more preferable that the organic solvent is not used at all.

The water-soluble organic solvent is preferably an organic solvent having a property of being miscible with water. Examples of the water-soluble organic solvent include alcohols such as methanol, ethanol, n-propanol, isopropanol, n-butanol, isobutanol, and t-butanol; glycols such as glycerin, ethylene glycol, and propylene glycol; and polyalkylene glycols such as polyethylene glycol. Among these, alcohols are preferred. The alcohol is preferably at least one selected from ethanol, methanol, 2-propanol, or t-butanol, more preferably at least one selected from ethanol, 2-propanol, or t-butanol, and still more preferably ethanol.

The ester-based organic solvent is preferably an ester obtained from a reaction of an organic acid and an alcohol. Examples of the ester-based organic solvent include ethyl acetate, methyl acetate, isopropyl acetate, t-butyl acetate, and methyl propionate, and at least one selected from ethyl acetate, isopropyl acetate, and methyl propionate is preferable, and ethyl acetate is more preferable.

A mixing ratio of the water-soluble organic solvent and the ester-based organic solvent can be, for example, 90:10 to 30:70 in terms of mass ratio, preferably 80:20 to 40:60, and more preferably 80:20 to 70:30. The mixed solvent of the water-soluble organic solvent and the ester-based organic solvent may further contain an aqueous solvent such as water or a buffer solution. The aqueous solvent can be added, for example, in a range of 1% to 30% by mass. A pH of the mixed solvent can be, for example, 3 to 10, and is preferably 4 to 9. The ester-based organic solvent may contain a physiologically active substance such as various drugs soluble in these solvents.

In a case where ethanol is used as the water-soluble organic solvent and ethyl acetate is used as the ester-based organic solvent, a mixing ratio of ethanol and ethyl acetate can be, for example, 80:20 to 70:30 in terms of mass ratio.

A concentration of the lipid is not particularly limited and can be appropriately adjusted, but can be set to 40 g/L to 250 g/L and is preferably 100 g/L to 200 g/L as a solution using a mixed solution of the water-soluble organic solvent and the ester-based organic solvent as a solvent.

In a case of producing the liposome, water (distilled water, water for injection, or the like), physiological saline, various buffer solutions or aqueous solutions of saccharides, and mixtures thereof (aqueous solvents) are preferably used as an aqueous solution (outer water phase) in which the liposome is dispersed. The buffer solution is not limited to organic and inorganic buffer solutions, and a buffer solution having a buffering action in the vicinity of a hydrogen ion concentration close to that of the body fluid is suitably used and examples thereof include a phosphate buffer solution, a Tris buffer solution, a citrate buffer solution, an acetate buffer solution, and a Good's buffer solution. A pH of the water phase can be, for example, 5 to 9, and is preferably 7 to 8. It is preferable to use a phosphate buffer solution (for example, pH = 7.4) as an aqueous solution (outer water phase) in which the liposome is dispersed. The inner water phase of the liposome may be an aqueous solution in which the liposomes are dispersed in a case of producing liposomes, or may be water, physiological saline, an aqueous solution of various buffer solutions or sugars, or a mixture thereof which is newly added. The water used as an outer water phase or an inner water phase is preferably free from impurities (dust, chemicals, or the like).

The physiological saline refers to an inorganic salt solution adjusted to be isotonic with the human body fluid, and may further have a buffering function. Examples of the physiological saline include saline containing 0.9 w/v% of sodium chloride, phosphate buffered saline (hereinafter, also referred to as PBS), and tris buffered saline.

In the step of mixing the dissolution solution of the gemcitabine hydrochloride obtained in the step (b) with the dispersion liquid of the empty liposome, the amount of water in the dispersion liquid is preferably 5 times or more and 15 times or less the amount of the gemcitabine hydrochloride, and more preferably 7 times or more and 13 times or less the amount of the gemcitabine hydrochloride.

The aqueous solution containing the liposome prepared through the emulsification step may be subjected to a post-treatment by a method such as centrifugal separation, ultrafiltration, dialysis, gel filtration, or freeze-drying in order to remove components that are not contained in the liposome or to adjust the concentration or the osmotic pressure.

The obtained liposomes can be made uniform in particle size by using dialysis, filtration, extrusion processing, or the like.

The extrusion refers to a step of passing liposomes through a filter having a fine pore to apply a physical shearing force, thereby carrying out microparticulation of the liposomes. In a case where the liposomes are passed through, rapid microparticulation thereof may be achieved by incubating the liposome dispersion liquid and the filter at a temperature higher than or equal to the phase transition temperature of the membrane constituting the liposome.

From the viewpoint of suitability for commercial production, it is preferable to prepare the empty liposome without performing the extrusion treatment, which is difficult in terms of manufacturing suitability (particularly, clogging or the like) and is costly.

### <Step (d)>

The step (d) is a step of adding an alkali to the dissolution solution of the gemcitabine hydrochloride obtained in the step (c) and adjusting the pH to be in a range of 6.8 to 7.8.

In a case where the pH is less than 6.8 or the pH exceeds 7.8, there is a problem in that the ester bond of the lipid constituting the liposome is easily hydrolyzed.

As the alkali in the step (d), a general water-soluble base such as sodium hydroxide and potassium chloride can be used, but the alkali is not particularly limited.

### <Step (e)>

The step (e) is a step of heating the dissolution solution obtained in the step (d) to 55°C or higher.

By heating to 55°C or higher, the gemcitabine hydrochloride is encapsulated in the liposome.

### <Step (f)>

The method for producing a liposome composition according to the embodiment of the present invention may further include, after the step (e), a step (f) of removing the gemcitabine hydrochloride present in the outer water phase. The step of removing the gemcitabine hydrochloride present in the outer water phase can be performed by dialysis. By replacing the outer water phase with a dialysis liquid, the unencapsulated gemcitabine hydrochloride and the solute present in the outer water phase can be removed.

### (Osmotic pressure adjusting step)

The method for producing a liposome composition according to the embodiment of the present invention may include, as desired, a step of adjusting the osmotic pressure of the inner water phase of the liposome to 2 times to 8 times the osmotic pressure of the outer water phase of the liposome.

In the step of adjusting the osmotic pressure, it is preferable to adjust the osmotic pressure of the inner water phase of the liposome to 2 times to 8 times the osmotic pressure of the outer water phase of the liposome. The osmotic pressure of the inner water phase of the liposome is more preferably adjusted to 2.5 times to 6 times and still more preferably adjusted to 3 times to 5 times the osmotic pressure of the outer water phase of the liposome.

By adjusting the osmotic pressure of the inner water phase and the outer water phase of the liposome, the leakage rate can be controlled. The step of adjusting the osmotic pressure is not particularly limited, and examples thereof include dialysis. In the method for producing a liposome composition according to the embodiment of the present invention, it is preferable to perform the step of encapsulating the gemcitabine hydrochloride in the liposome and the step of adjusting the osmotic pressure at the same time from the viewpoint of production efficiency.

The liquid obtained after the step of encapsulating the gemcitabine hydrochloride in the liposome has a uniform solute in the outer water phase and the inner water phase, and the osmotic pressure at this time can be defined as the osmotic pressure of the inner water phase of the completed liposome composition. However, in the subsequent step of adjusting the osmotic pressure by dialysis of the outer water phase, the heating operation is limited to a case where the solute in the inner water phase is sufficiently retained, for example, by suppressing the heating operation to the phase transition of the lipid or lower. In addition, the osmotic pressure of the outer water phase can be defined by the osmotic pressure of a dialysis liquid used in the final dialysis step. However, it is limited to a case where the outer water phase can be sufficiently replaced with a dialysis liquid. In addition, for the complete liquid of the liposome composition, the osmotic pressure of the inner water phase and the outer water phase can be obtained by quantifying the composition concentration of the solute in the outer water phase and the composition concentration of the solute in the inner water phase using centrifugal separation or ultrafiltration, and measuring the osmotic pressure of the composition liquid.

It suffices that the osmotic pressure is measured according to the osmotic pressure measuring method described in the Japanese Pharmacopoeia, Sixteenth Edition. Specifically, the osmolality can be determined by measuring a degree of freezing point (ice point) depression of water. Further, the degree of the drop of the solidifying point of water is defined **in terms of a** molar concentration of a solute, **and thus** the osmolar concentration can be also determined from the molar concentration of the solute.

The osmotic pressure of the outer water phase has an important influence on the living body at the time of administration. In a case where it is far from the osmotic pressure of the body fluid, hemolysis and pain due to the movement of water in each tissue occur. Therefore, the osmotic pressure of the outer water phase is preferably 200 mOsmol/L to 400 mOsmol/L, more preferably 250 mOsmol/L to 350 mOsmol/L, and still more preferably isotonic with the body fluid.

### <Evaporation step>

In the method for producing a liposome composition, an evaporation step may be provided as necessary. In the evaporation step, the organic solvent is evaporated from the aqueous solution containing liposomes obtained in the emulsifying step. In the present invention, the evaporation step includes at least one of a step of forcibly removing a part or all of the organic solvent derived from the oil phase as the evaporation step, or a step of naturally evaporating a part or all of the organic solvent in the oil phase in the process of stirring and emulsification.

The method of evaporating the organic solvent in the evaporation step is not particularly limited, and for example, at least one of a step of evaporating the organic solvent by heating, a step of continuing to stand or gently stirring after emulsification, or a step of performing vacuum deaeration may be performed.

In the step of evaporating the organic solvent, it is preferable that the concentration of the organic solvent contained in the aqueous solution containing the liposome is set to 15% by mass or less within 30 minutes after the start of the step of evaporating the organic solvent.

### <Sterile filtration>

It is preferable that the liposome composition is subjected to sterile filtration. Regarding the filtration method, it is possible to remove unwanted materials from an aqueous solution containing liposomes by using a hollow fiber membrane, a reverse osmosis membrane, a membrane filter, or the like. In the present invention, it is preferable to filter the liposome composition through a filter having a sterilizable pore size (preferably a 0.2 µm filtration sterilization filter).

To prevent an effect of deformation of liposomes on the average particle size, the sterile filtration step and the below-described aseptic filling step are preferably carried out at a temperature lower than or equal to the phase transition temperature of the lipid constituting the liposome. For example, in a case where the phase transition temperature of the lipid is around 50°C, the sterile filtration step and the below-described aseptic filling step are carried out at temperature of preferably about 0°C to 40°C, and more specifically about 5°C to 30°C.

### <Aseptic filling>

The liposome composition obtained after sterile filtration is preferably aseptically filled for medical applications. Known methods can be applied for aseptic filling. A liposome composition suitable for medical applications can be prepared by aseptically filling the liposome composition in a container.

### (Use of liposome composition)

The liposome composition can be supplied in a state of being filled in a container. The container in which the liposome composition according to the embodiment of the present invention is filled is not particularly limited, and it is preferably made out of a material having low oxygen permeability. Examples of the container include a plastic container, a glass container, and a laminated film bag with an aluminum foil, an aluminum vapor deposition film, an aluminum oxide vapor deposition film, a silicon oxide vapor deposition film, a polyvinyl alcohol, an ethylene-vinyl alcohol copolymer, a polyethylene terephthalate, a polyethylene naphthalate, a polyvinylidene chloride, or the like as a gas barrier layer. The container can be shielded from light by employing, for example, a bag using a colored glass, an aluminum foil, an aluminum vapor deposition film, or the like, if necessary.

In the container in which the liposome composition is filled, in order to prevent oxidation due to oxygen existing in the space inside the container, it is preferable to replace the gas in the container space and drug solution with an inert gas such as nitrogen. For example, an injection solution is bubbled with nitrogen, whereby the filling of the injection solution into a container can be carried out under a nitrogen atmosphere.

As a route of administration of the liposome composition, parenteral administration is preferable. Examples of the parenteral administration include intravenous injection such as intravenous drip, intramuscular injection, intraperitoneal injection, subcutaneous injection, intraocular injection, and intrathecal injection. Administration methods include administration by syringe or drip infusion.

The dose and the number of times of administration of the drug contained in the liposome composition are selected in a range of 0.01 mg/kg to 100 mg/kg per day. However, the liposome composition according to the embodiment of the present invention is not limited to these doses.

The tumor and the cancer in which the liposome composition can be effectively used are not particularly limited, and examples thereof include breast cancer, uterine corpus cancer, ovarian cancer, prostate cancer, lung cancer, gastric (gastric gland) cancer, non-small cell lung cancer, pancreatic cancer, head and neck squamous cell carcinoma, esophageal cancer, bladder cancer, melanoma, colorectal cancer, renal cell carcinoma, non-Hodgkin's lymphoma, and urinary tract urothelial carcinoma, and pancreatic cancer is preferable.

### Examples

Hereinafter, the present invention will be described in detail with reference to Examples. However, the present invention is not limited to Examples.

The osmotic pressure is calculated from the molar concentration of the solute.

The average particle diameter and the particle size distribution are measured by diluting a sample with phosphate buffered saline to 33 times the mass and measuring the volume average particle diameter and the volume distribution of the particle diameter by a dynamic light scattering method using FPAR-1000AS (manufactured by Otsuka Electronics Co., Ltd.).

Gemcitabine encompassed in the liposome is quantified by liquid chromatography/ultraviolet-visible absorbance detection. The measurement conditions were as follows.
Measurement wavelength: 272 nm
Column: Waters Atlantis T3φ, 4.6 mm × 150 mm, 5 µm (manufactured by Waters Corporation)
Column temperature: constant temperature of around 40°C
Both the mobile phases A and B are a mixed solution of water/methanol/trifluoroacetic acid, and the feeding of the mobile phases is controlled by changing the mixing ratio of the mobile phases A and B to control the concentration gradient.
Mobile phase A: methanol/water volume ratio of 5/95, trifluoroacetic acid of 0.1%
Mobile phase B: methanol/water volume ratio of 25/75, trifluoroacetic acid of 0.1%
Flow rate: 1.0 mL per minute
Injection volume: 10 µL
Liquid temperature at time of sample injection: constant temperature around 25°C

The lipid constituting the liposome was quantified by liquid chromatography/CAD detection. The measurement conditions were as follows.
Column: Waters Xbridge, 4.6 mm × 250 mm, 5 µm (manufactured by Waters Corporation)
Column temperature: constant temperature of around 40°C

As the mobile phases A and B, both were 7 mmol/L ammonium acetate methanol solutions.
Flow rate: 1.0 mL
Injection volume: 10 µL
Liquid temperature at time of sample injection: constant temperature around 25°C

The lysophospholipid contained in the lipid constituting the liposome was quantified by the following method.

10 mg of C17 lysophosphatidylcholine was weighed in a 10 mL volumetric flask, and the flask was filled up with ethanol. This solution was further diluted 50 times to obtain an internal standard solution.

20 µL of the liposome composition was weighed, and 380 µL of ethanol was added thereto to perform 20-fold dilution. 400 µL of the internal standard solution was added to this solution and mixed therewith to obtain a measurement sample, and the measurement was performed by liquid chromatography mass spectrometry. The measurement conditions were as follows.
Column: ACQUITY UPLC BEH C18, 2.1 mm × 50 mm, 1.7 µm (manufactured by Waters Corporation)
Column temperature: constant temperature of around 40°C
Mobile phase: The concentration gradient was controlled by changing the mixing ratio of the mobile phases A and B.
Mobile phase A: water/methanol volume ratio of 20/80
Mobile phase B: methanol
Flow rate: 0.5 mL per minute
Injection amount: 5 µL
Liquid temperature at time of sample injection: constant temperature around 25°C

### (Example 1)

### <Preparation of oil phase>

395 g, 49 g, and 102 g of hydrogenated soybean phosphatidylcholine, cholesterol, and N-(carbonyl-methoxypolyethylene glycol 2000)-1,2-distearoyl-sn-glycero-3-phosphoethanolamine sodium salt (hereinafter, also referred to as DSPE-PEG) were weighed to have a molar ratio of 76/19/5, 5.7 kg of ethanol and 2.2 kg of ethyl acetate were added thereto, and the mixture was heated to 70°C to dissolve the lipids, thereby obtaining an oil phase.

### <Preparation of water phase>

4 mmol/L phosphate buffer solution was prepared and used as a water phase.

### <Preparation of drug-unencapsulated liposome>

The water phase was heated to 70°C, the oil phase was added thereto such that the volume ratio of water phase/oil phase was 8/3, and the mixture was mixed for 30 minutes at a circumferential speed of 20 m/s and 7,000 rpm using a rotary stirring emulsifier (manufactured by M-TECHNICS Co., Ltd.). Thereafter, the organic solvent and water were evaporated by supplying nitrogen while heating the mixture to a temperature equal to or higher than the phase transition temperature. Next, the entire amount of the obtained liquid was dialyzed and replaced with a 0.9% sodium chloride solution, and the volume was adjusted to be about 1/10 of the volume before emulsification, thereby obtaining a drug-unencapsulated liposome (empty liposome). The average particle diameter at this time was 63.9 nm.

### (a) Step of preparing a dissolution solution of gemcitabine hydrochloride

Gemcitabine hydrochloride was used as a drug. Gemcitabine hydrochloride was purchased from TEVA Pharmaceutical Industries Ltd.

### (1) Step of adding gemcitabine hydrochloride to water

In order to prevent the powder of gemcitabine hydrochloride having high pharmacological activity from being exposed to the operator, the following work was performed in an isolator that could completely isolate the work from the outside. 305 g of gemcitabine hydrochloride was put into a plastic bag with an extension tube connected to the outside of the isolator using a burette. In this case, the extension tube was closed by a pinch valve. 1,452 g of water for injection at room temperature was put into the plastic bag to wash the burette, and gemcitabine hydrochloride was dispersed. A tube extended from a sealed tank (hereinafter, a loading tank) made of SUS316L with a temperature control jacket installed outside the isolator and the plastic bag was connected, and the gemcitabine hydrochloride aqueous dispersion liquid was put into the loading tank. A solution consisting of 17.0 g of disodium hydrogen phosphate dihydrate, 2.7 g of sodium dihydrogen phosphate dihydrate, and 1,106.6 g of water for injection was put into the loading tank from the plastic bag to wash out the gemcitabine hydrochloride remaining in the plastic bag, the extension tube, and the like. By cutting the extension tube while pinching and crimping the extension tube, the gemcitabine hydrochloride of the highly pharmaceutically active substance could be safely put into the loading tank without scattering from the isolator or the loading tank.

Next, the gemcitabine hydrochloride was well dispersed in the loading tank by rotating a stirring blade having a diameter of 70 mm at 400 rpm (88 m/min). In this stirring, the rotation direction of the stirring blade was set such that the direction of the flow of the liquid generated by the stirring blade was a direction from the stirring blade portion toward the upper portion of the stirring blade shaft.

### (2) Step of raising the temperature to a range of 50°C or higher and 60°C or lower and stirring (step of dissolving gemcitabine hydrochloride)

While maintaining the above-described stirring, the jacket of the loading tank was adjusted, and the temperature control jacket medium of the loading tank was raised from room temperature to 56°C to dissolve the gemcitabine hydrochloride. After about 15 minutes after the temperature rise, a small window installed on the lid of the loading tank was temporarily opened, and it was visually confirmed that the gemcitabine hydrochloride was completely dissolved. The liquid temperature of the gemcitabine hydrochloride aqueous solution at that time was 54°C.

### (3) Step of adding an alkali to adjust the pH to a range of 3.2 to 4.6

8 mol/L of sodium hydroxide was added to the loading tank to adjust the pH to 3.6.

### (b) Step of adding sodium chloride and step of lowering the temperature to lower than 50°C

A sodium chloride aqueous solution obtained by dissolving 96.9 g of sodium chloride in 400 g of water for injection was subsequently put into the loading tank. Thereafter, the jacket temperature was lowered to set the liquid temperature to 45°C. At this point, it was confirmed that the gemcitabine hydrochloride was satisfactorily dissolved.

### (c) Step of mixing the dissolution solution of the gemcitabine hydrochloride with the dispersion liquid of the empty liposome

Subsequently, 3.2 kg of the empty liposome kept at 45°C described in (b) above was put into the loading tank. A sodium chloride aqueous solution obtained by dissolving 96.9 g of sodium chloride in 400 g of water for injection was subsequently put into the loading tank. Thereafter, the liquid temperature was maintained at 45°C by the jacket.

### (d) Step of adding an alkali to adjust the pH to be in a range of 6.8 to 7.8

8 mol/L of sodium hydroxide was added to the loading tank to adjust the pH to 6.0. This pH adjustment work was performed within about 5 minutes after the empty liposome was put into the loading tank. Further, 8 mol/L of sodium hydroxide was added little by little, and the pH was finally finely adjusted to 7.2. The time required for the fine adjustment of the pH was about 20 minutes.

### (e) Step of heating the dissolution solution to 55°C or higher

The dissolution solution obtained in the step (d) was heated from 45°C to 72°C over about 25 minutes. Thereafter, the temperature was maintained at about 72°C for 10 minutes, and then the temperature was lowered to 45°C over about 15 minutes or more.

### (f) Step of removing the gemcitabine hydrochloride present in the outer water phase

The dissolution solution obtained in the step (e) was diluted with 29.8 kg of a 1,016 mmol/L sucrose/37 mmol/L histidine solution. Next, the total amount of the obtained liquid was dialyzed with a solution adjusted with 469 g of histidine, 28.4 kg of sucrose, and 263 kg of water for injection. By dialysis and replacement, the unencapsulated gemcitabine hydrochloride and each solute present in the outer water phase of the drug loading solution were removed, and the outer water phase was replaced with the dialysis liquid. The obtained liquid was used as a liposome finished liquid. The osmotic pressure determined from the molar concentration of the solute of this liquid was 361 mOsm/L. By the above-described steps, a gemcitabine-encapsulating liposome composition having a gemcitabine hydrochloride concentration of 0.98 mg/mL, an average particle diameter of 72.1 nm, an inner water phase osmotic pressure of 1,052 mOsm/L, an outer water phase osmotic pressure of 361 mOsm/L, and an osmotic pressure of the inner water phase to the outer water phase of 2.9 times was obtained. The lysophospholipid contained in the lipid constituting the liposome was 0.37 mol% with respect to the total amount of the phospholipids other than the lysophospholipid contained in the lipid constituting the liposome.

### (Comparative Example 1)

The steps of <Preparation of oil phase>, <Preparation of water phase>, and <Preparation of drug-free liposomes> were carried out in the same manner as in Example 1.

### (a) Step of preparing gemcitabine hydrochloride solution

Gemcitabine hydrochloride was used as a drug. Gemcitabine hydrochloride was purchased from TEVA Pharmaceutical Industries Ltd.

### (1) Step of adding gemcitabine hydrochloride to water

304 g of gemcitabine hydrochloride was put into a plastic bag with an extension tube connected to the outside of the isolator using a burette. In this case, the extension tube was closed by a pinch valve. 1,456 g of water for injection at room temperature was put into the plastic bag to wash the burette, and the gemcitabine hydrochloride was dispersed. A tube extended from a sealed tank (hereinafter, a loading tank) made of SUS316L with a temperature control jacket installed outside the isolator and the plastic bag was connected, and the gemcitabine hydrochloride aqueous dispersion liquid was put into the loading tank. A solution consisting of 17.0 g of disodium hydrogen phosphate dihydrate, 2.7 g of sodium dihydrogen phosphate dihydrate, 97.0 g of sodium chloride, and 965 g of water for injection was put into the loading tank from the plastic bag to wash out the gemcitabine hydrochloride remaining in the plastic bag, the extension tube, and the like. By cutting the extension tube while pinching and crimping the extension tube, the gemcitabine hydrochloride of the highly pharmaceutically active substance could be safely put into the loading tank without scattering from the isolator or the loading tank. Next, the gemcitabine hydrochloride was dispersed in the loading tank by rotating a stirring blade having a diameter of 70 mm at 200 rpm. In this stirring, the rotation direction of the stirring blade was set such that the direction of the flow of the liquid generated by the stirring blade was a direction from the upper part of the stirring blade shaft toward the stirring blade portion.

### (2) Step of dissolving gemcitabine hydrochloride

While maintaining the above-described stirring, the jacket of the loading tank was adjusted, and the temperature control jacket medium of the loading tank was adjusted from room temperature to 56°C to attempt to dissolve the gemcitabine hydrochloride. The solution condition was a condition in which the dissolution easily proceeded in a small scale. However, in this production scale, the gemcitabine hydrochloride stuck to the bottom of the tank, and the dissolution did not proceed at all. 8.0 mol/mL of sodium hydroxide was added to adjust the pH to 3.6 in the next step in a state where the gemcitabine hydrochloride could not be dissolved, but the pH increased significantly because the gemcitabine hydrochloride could not be dissolved, and even after 1 hour, the complete dissolution could not be confirmed. It was considered that the dissolution could be promoted by largely opening the lid of the tank and physically breaking the gemcitabine hydrochloride fixed to the bottom of the tank, but the lid of the tank could not be largely opened to ensure the safety of the operator. Since the dissolution could not be completed in the desired time, it was not possible to complete the liposome encapsulating the drug by giving up continuing the production.

### (Reference Example 1) Measurement of saturated solubility of gemcitabine hydrochloride

### (1) Measurement of solubility of gemcitabine hydrochloride in absence of sodium chloride imitating Example 1

0.95 g of disodium hydrogen phosphate dihydrate, 0.15 g of sodium dihydrogen phosphate dihydrate, and 51.1 g of water were put into a 100 mL plastic container and dissolved to produce a buffer containing no sodium chloride.

3.4 g of gemcitabine hydrochloride was put into 5.8 g of this buffer and 8.1 g of water, and the mixture was stirred at 55°C for 2 hours. 5 mL of this supernatant was collected, and the filtrate obtained by filtration was used as a sample solution.

This sample solution was quantified by high-performance liquid chromatography (HPLC) at a detection wavelength of 272 nm, and the saturated solubility of 156 mg/mL in the absence of sodium chloride was obtained.

It was found that, in the blending amount in the production step of Example 1, it is necessary to dissolve at 119 mg/mL, but the saturated solubility determined experimentally was higher than this value, and the conditions were sufficient for dissolution.

### (2) Measurement of solubility of gemcitabine hydrochloride in presence of sodium chloride imitating Comparative Example 1

0.95 g of disodium hydrogen phosphate dihydrate, 0.15 g of sodium dihydrogen phosphate dihydrate, 5.4 g of sodium chloride, and 67.7 g of water were put into a 100 mL plastic container and dissolved to produce a buffer containing sodium chloride.

3.4 g of gemcitabine hydrochloride was put into 7.4 g of this buffer and 8.1 g of water, and the mixture was stirred at 55°C for 2 hours. 5 mL of this supernatant was collected, and the filtrate obtained by filtration was used as a sample solution.

This sample solution was quantified by HPLC at a detection wavelength of 272 nm, and the saturated solubility of 90 mg/mL in the presence of sodium chloride was obtained.

It was found that, in the blending amount in the production step of Comparative Example 1, it is necessary to dissolve at 126 mg/mL, but the saturated solubility determined experimentally was lower than this value, and the conditions were not sufficient for dissolution.

In a case of producing a gemcitabine hydrochloride solution at a small scale, the saturated solubility was exceeded by the water brought in by the pH adjustment step to pH 3.6, which is a subsequent step, and the solution could be completely dissolved. However, in the actual commercial production scale required in Comparative Example 1, adhesion to the bottom surface of the tank occurs, and the solution cannot be dissolved within a realistic production time. It was found that this was because the blending amount exceeding the saturated solubility was set as shown in the present reference example.

## Claims

1. A method for producing a liposome composition, the liposome composition containing a liposome containing an inner water phase and gemcitabine hydrochloride in a dissolved state, and an outer water phase which is an aqueous solution dispersing the liposome, the method comprising: steps (a) to (e) below:
step (a) of preparing a dissolution solution of gemcitabine hydrochloride, the step including steps (1) to (3) below;
step (1) of adding gemcitabine hydrochloride to water,
step (2) of raising a temperature of the mixture obtained in the step (1) within a range of 50°C or higher and 60°C or lower and stirring the mixture, and
step (3) of adding an alkali to the mixture obtained in the step (2) to adjust a pH to within a range of 3.2 to 4.6,
step (b) of adding sodium chloride to the dissolution solution of gemcitabine hydrochloride obtained in the step (a), and a step of lowering the temperature of the obtained mixture to less than 50°C;
step (c) of mixing the dissolution solution of gemcitabine hydrochloride obtained in the step (b) with a dispersion liquid of empty liposomes;
step (d) of adding an alkali to the dissolution solution of the gemcitabine hydrochloride obtained in the step (c) and adjusting a pH to be in a range of 6.8 to 7.8; and
step (e) of heating the dissolution solution obtained in the step (d) to 55°C or higher.

2. The method for producing a liposome composition according to claim 1, wherein the step (a) is a step of preparing a gemcitabine hydrochloride solution in which gemcitabine hydrochloride is completely dissolved.

3. The method for producing a liposome composition according to claim 1, wherein the step (a) is a step of preparing a gemcitabine hydrochloride solution in which the gemcitabine hydrochloride is completely dissolved by completion of step (2).

4. The method for producing a liposome composition according to claim 1, wherein a stirring condition in the step (2) of the step (a) is such that a direction of a flow of a liquid generated by a stirring blade is a direction from a stirring blade portion toward an upper portion of a stirring blade shaft, and a circumferential speed of stirring is 10 to 300 m/min.

5. The method for producing a liposome composition according to any one of claims 1 to 4, further comprising: step (f) of removing gemcitabine hydrochloride present in the outer water phase after the step (e).

6. The method for producing a liposome composition according to any one of claims 1 to 4, wherein in the step (1) of the step (a) of adding gemcitabine hydrochloride to water, an amount of water added is 5 times or more and 15 times or less an amount of the gemcitabine hydrochloride.

7. The method for producing a liposome composition according to any one of claims 1 to 4, wherein in the step of mixing the dissolution solution of the gemcitabine hydrochloride obtained in the step (b) with the dispersion liquid of the empty liposome in the step (c), an amount of water in the dispersion liquid is 5 times or more and 15 times or less an amount of the gemcitabine hydrochloride.

8. A liposome composition obtained by a production method comprising: the steps (a) to (e) below, wherein the liposome composition includes liposome having an inner water phase and an aqueous solution constituting an outer water phase in which the liposomes are dispersed, and the liposomes encapsulate gemcitabine hydrochloride in a dissolved state.
step (a) of preparing a dissolution solution of gemcitabine hydrochloride, the step including steps (1) to (3) below;
step (1) of adding gemcitabine hydrochloride to water,
step (2) of raising a temperature of the mixture obtained in the step (1) within a range of 50°C or higher and 60°C or lower and stirring the mixture, and
step (3) of adding an alkali to the mixture obtained in the step (2) to adjust a pH to within a range of 3.2 to 4.6,
step (b) of adding sodium chloride to the dissolution solution of gemcitabine hydrochloride obtained in the step (a), and a step of lowering the temperature of the obtained mixture to less than 50°C;
step (c) of mixing the dissolution solution of gemcitabine hydrochloride obtained in the step (b) with a dispersion liquid of empty liposomes;
step (d) of adding an alkali to the dissolution solution of the gemcitabine hydrochloride obtained in the step (c) and adjusting a pH to be in a range of 6.8 to 7.8; and
step (e) of heating the dissolution solution obtained in the step (d) to 55°C or higher.

9. The liposome composition according to claim 8, wherein the step (a) is a step of preparing a gemcitabine hydrochloride solution in which the gemcitabine hydrochloride is completely dissolved.

10. The liposome composition according to claim 8, wherein the step (a) is a step of preparing a gemcitabine hydrochloride solution in which the gemcitabine hydrochloride is completely dissolved by completion of step (2).

11. The liposome composition according to claim 8, wherein a stirring condition in the step (2) of the step (a) is such that a direction of a flow of a liquid generated by a stirring blade is a direction from a stirring blade portion toward an upper portion of a stirring blade shaft, and a circumferential speed of stirring is 10 to 300 m/min.

12. The liposome composition according to any one of claims 8 to 11, further comprising: step (f) of removing gemcitabine hydrochloride present in the outer water phase after the step (e).

13. The liposome composition according to any one of claims 8 to 11, wherein in the step (1) of the step (a) of adding gemcitabine hydrochloride to water, an amount of water added is 5 times or more and 15 times or less an amount of the gemcitabine hydrochloride.

14. The liposome composition according to any one of claims 8 to 11, wherein in the step of mixing the dissolution solution of the gemcitabine hydrochloride obtained in the step (b) with the dispersion liquid of the empty liposome in the step (c), an amount of water in the dispersion liquid is 5 times or more and 15 times or less an amount of the gemcitabine hydrochloride.
